(19) Europäisches Patentamt / European Patent Office / Office européen des brevets

(11) **EP 4 306 533 A1**

(12) **EUROPEAN PATENT APPLICATION**
published in accordance with Art. 153(4) EPC

(43) Date of publication:
**17.01.2024 Bulletin 2024/03**

(21) Application number: **22766399.4**

(22) Date of filing: **11.03.2022**

(51) International Patent Classification (IPC):
*C07K 5/083* (2006.01)      *A61K 38/06* (2006.01)
*A61K 38/07* (2006.01)      *A61P 25/16* (2006.01)
*A61P 9/10* (2006.01)       *A61P 7/04* (2006.01)
*C07K 7/06* (2006.01)       *C07K 5/103* (2006.01)
*A61K 38/08* (2019.01)      *A61P 25/28* (2006.01)
*A61P 25/00* (2006.01)

(52) Cooperative Patent Classification (CPC):
Y02P 20/55

(86) International application number:
**PCT/CN2022/080445**

(87) International publication number:
**WO 2022/188877 (15.09.2022 Gazette 2022/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **11.03.2021 CN 202110266149**

(71) Applicant: **Wuhan Innerse Pharmaceutical Inc.
Wuhan, Hubei 430075 (CN)**

(72) Inventor: **LI, Jianxiong
Wuhan, Hubei 430075 (CN)**

(74) Representative: **Høiberg P/S
Adelgade 12
1304 Copenhagen K (DK)**

(54) **SYNTHETIC PEPTIDE AND APPLICATION THEREOF**

(57) The present invention relates to the technical field of medicines. Disclosed are a synthetic peptide and an application thereof. The chemical formula of the synthetic peptide is (Gly)x-(beta-Ala-L-His)y-(Gly)z, (Gly)x-(beta-Ala-L-1-Methyl-His)y-(Gly)z or (Gly)x-(beta-Ala-L-3-Methyl-His)y-(Gly)z; y being equal to or greater than 1, and x and z being 0-3 and being not both equal to zero. In the synthetic peptide in the present invention, glycine, carnosine, anserine, ophidine or the like are creatively combined, and the developed mosaic synthetic peptide unexpectedly penetrates the blood-brain barrier to enter the brain by means of intravenous injection and effectively plays a role in treating nervous system diseases. In the present invention, a remarkable treatment effect can be achieved using only 3 mg/kg of synthetic polypeptide intravenous injection dosage.

EP 4 306 533 A1

## Description

## Technical field

The invention belongs to the technical field of medicine, and in particular relates to a synthetic peptide and application thereof.

## Background

Glycine is the amino acid with the simplest chemical structure. Despite its simple structure, glycine is an important inhibitory neurotransmitter in the central nervous system, playing an important role in controlling neuronal excitability. It has been found in animal experiments that glycine has significant neuroprotective effects, and it was also found in some clinical trials that glycine can improve cognitive dysfunction and dementia.

Carnosine (L-Carnosine) is a dipeptide composed of two types of amino acids, β-alanine and L-histidine. Carnosine has strong antioxidant capacity and is beneficial to human body. Carnosine has been shown to clear off reactive oxygen species (ROS) and α-β-unsaturated aldehydes formed during oxidative stress by the excessive oxidation of fatty acids on cell membrane. Carnosine has anti-inflammatory, anti-glycation, anti-oxidative and chelating function, and it can be used as a nonprescription food supplement and it is promising in the prevention and auxiliary treatment of chronic diseases such as cardiovascular diseases and neurodegenerative diseases. The neuroprotective mechanism of carnosine can prevent permanent cerebral ischemia in animal experiments. Carnosine is also an important intracellular antioxidant. Carnosine is not only non-toxic, but also has strong anti-oxidative effect, so it has attracted widespread attention as a new food additive and pharmaceutical reagent. Carnosine participates in the peroxidation reaction in the cell. In addition to inhibiting the peroxidation process on cell membrane, carnosine can also inhibit the relevant peroxidation reactions within the cell. In 1900, Gulewitsch from Russia first discovered carnosine, and then scientists from various countries extracted and isolated other histidine dipeptide derivatives from different types of muscle tissue, such as anserine, which is a dipeptide composed of β-alanine and 1-methyl-L-histidine, and balenine (also known as ophidine), which is composed of β-alanine and 3-methyl-L-histidine. The amount of these carnosine and carnosine derivatives vary in different animals, and the amount and ratio of these histidine dipeptides are also different among different species, with certain specificity. Not only present in muscle tissue, these histidine dipeptides are also found in other types of tissue, such as brain tissue. These carnosine derivatives are water-soluble and have strong and significant anti-oxidative, anti-aging, and uric acid-lowering functions. They have been used as natural antioxidants and uric acid-lowering diet therapy in food industry, and they also have certain neuroprotective functions.

Although glycine, carnosine and carnosine derivatives all have certain protective effects for brain, they often require relatively large doses, for example, the glycine dosage for peripheral administration is up to 800mg/kg, which can lead to serious side effects, and it is also inconvenient to use. As a result, their practical clinical applications are limited. The effect of using carnosine, anserine, or ophidine alone is not good neither, which is very different from the therapeutic effect of NA-1, the latest drug.

## Summary

The present invention relates to a synthetic peptide consisting of some of glycine, beta-alanine, histidine, 1-methylhistidine and 3-methylhistidine, especially glycine, carnosine, anserine and even ophidine, so as to form a polypeptide compound with both the structural characteristics of carnosine, anserine or ophidine as well as the structural characteristics of glycine. Surprisingly, the polypeptide can easily pass the blood-brain barrier, and exhibit good biological activity through intravenous administration, thus is promising in the treatment of neurological diseases, especially brain damage and stroke. The technical embodiments of the present invention are described below.

In one aspect, the present invention provides a synthetic peptide with the following formula:

(Gly)x-(beta-Ala-His)y-(Gly)z

or

(Gly)x-(beta-Ala-1-Methyl-His)y-(Gly)z

or

(Gly)x-(beta-Ala-3-Methyl-His)y-(Gly)z;

in the formula, x is an integer between 0-3 (such as 0, 1, 2 and 3), y is an integer between 1-3 (such as 1, 2 and 3), z is an integer between 0-3 (such as 0, 1, 2, and 3), and x and z are not both equal to zero.

Wherein, the amino acids in the present invention are abbreviated as follows:

Glycine: Gly; beta-alanine: β-Ala or beta-Ala; L-histidine: His; 1-methylhistidine: 1-Methyl-His or (1-Methyl-His); 3-methylhistidine: 3-Methyl-His or (3-Methyl-His).

Wherein, the chemical formula (Gly)x-(beta-Ala-His)y-(Gly)z may be selected from any of:

(a) beta-Ala-His-Gly;
(b) Gly-beta-Ala-His;
(c) beta-Ala-His-Gly-Gly;
(d) Gly-Gly-beta-Ala-His;
(e) Gly-beta-Ala-His-Gly;
(f) Gly-Gly-beta-Ala-His-Gly-Gly;
(g) beta-Ala-His-beta-Ala-His-Gly;
(h) Gly-beta-Ala-His-beta-Ala-His;
(i) beta-Ala-His-beta-Ala-His-Gly-Gly;
(j) Gly-Gly-beta-Ala-His-beta-Ala-His;
(k) Gly-beta-Ala-His-beta-Ala-His-Gly;
(l) Gly-Gly -beta-Ala-His-beta-Ala-His-beta-Ala-His-Gly;
(m) beta-Ala-His-beta-Ala-His-beta-Ala-His-Gly-Gly-Gly;
(n) Gly-Gly-Gly-beta-Ala-His-beta-Ala-His-beta-Ala-His;
(o) Gly-beta-Ala-His-beta-Ala-His-beta-Ala-His-Gly-Gly.

Wherein, the chemical formula (Gly)x-(beta-Ala-1-Methyl-His)y-(Gly)z may be selected from any of:

(a) beta-Ala-(1-Methyl-His)-Gly;
(b) Gly-beta-Ala-(1-Methyl-His);
(c) beta-Ala-(1-Methyl-His)-Gly-Gly;
(d) Gly-Gly-beta-Ala-(1-Methyl-His);
(e) Gly-beta-Ala-(1-Methyl-His)-Gly;
(f) Gly-Gly-beta-Ala-(1-Methyl-His)-Gly-Gly;
(g) beta-Ala-(1-Methyl-His)-beta-Ala-(1-Methyl-His)-Gly;
(h) Gly-beta-Ala-(1-Methyl-His)-beta-Ala-(1-Methyl-His);
(i) beta-Ala-(1-Methyl-His)-beta-Ala-(1-Methyl-His)-Gly-Gly;
(j) Gly-Gly-beta-Ala-(1-Methyl-His)-beta-Ala-(1-Methyl-His);
(k) Gly-beta-Ala-(1-Methyl-His)-beta-Ala-(1-Methyl-His)-Gly;
(l) Gly-Gly-beta-Ala-(1-Methyl-His)-beta-Ala-(1-Methyl-His)-beta-Ala-(1-Methyl-His)-Gly;
(m) beta-Ala-(1-Methyl-His)-beta-Ala-(1-Methyl-His)-beta-Ala-(1-Methyl-His)-Gly-Gly-Gly;
(n) Gly-Gly-Gly-beta-Ala-(1-Methyl-His)-beta-Ala-(1-Methyl-His)-beta-Ala-(1-Methyl-His);
(o) Gly-beta-Ala-(1-Methyl-His)-beta-Ala-(1-Methyl-His)-beta-Ala-(1-Methyl-His)-Gly-Gly.

Wherein, the chemical formula (Gly)x-(beta-Ala-3-Methyl-His)y-(Gly)z may be selected from any of:

(a) beta-Ala-(3-Methyl-His)-Gly;
(b) Gly-beta-Ala-(3-Methyl-His);
(c) beta-Ala-(3-Methyl-His)-Gly-Gly;
(d) Gly-Gly-beta-Ala-(3-Methyl-His);
(e) Gly-beta-Ala-(3-Methyl-His)-Gly;
(f) Gly-Gly-beta-Ala-(3-Methyl-His)-Gly-Gly;
(g) beta-Ala-(3-Methyl-His)-beta-Ala-(3-Methyl-His)-Gly;
(h) Gly-beta-Ala-(3-Methyl-His)-beta-Ala-(3-Methyl-His);
(i) beta-Ala-(3-Methyl-His)-beta-Ala-(3-Methyl-His)-Gly-Gly;
(j) Gly-Gly-beta-Ala-(3-Methyl-His)-beta-Ala-(3-Methyl-His);
(k) Gly-beta-Ala-(3-Methyl-His)-beta-Ala-(3-Methyl-His)-Gly;
(l) Gly-Gly-beta-Ala-(3-Methyl-His)-beta-Ala-(3-Methyl-His)-beta-Ala-(3-Methyl-His)-Gly;
(m) beta-Ala-(3-Methyl-His)-beta-Ala-(3-Methyl-His)-beta-Ala-(3-Methyl-His)-Gly-Gly-Gly;
(n) Gly-Gly-Gly-beta-Ala-(3-Methyl-His)-beta-Ala-(3-Methyl-His)-beta-Ala-(3-Methyl-His);
(o) Gly-beta-Ala-(3-Methyl-His)-beta-Ala-(3-Methyl-His)-beta-Ala-(3-Methyl-His)-Gly-Gly.

Preferably, the chemical formula of the synthetic peptide of the present invention may be selected from any of:

(a) beta-Ala-His-Gly;
(b) Gly-beta-Ala-His;
(c) beta-Ala-His-Gly-Gly;
(d) Gly-Gly-beta-Ala-His;
(e) Gly-beta-Ala-His-Gly;
(f) Gly-Gly-beta-Ala-His-Gly-Gly.

More preferably, the chemical formula of the synthetic peptide of the present invention may be selected from any of:

(a)beta-Ala-L-His-Gly;
(b)Gly-beta-Ala-L-His.

In another aspect, the present invention also provides the aforementioned synthetic peptide or its salt for use in medicine. The present invention also provides the aforementioned multifunctional polypeptide or its salt for treating neurological diseases. The present invention also provides a method for treating neurological diseases by using the aforementioned multifunctional polypeptide or its salt.

Specifically, the neurological disease may be ischemic stroke, hemorrhagic stroke, cerebral trauma, Alzheimer's disease, Parkinson's disease or other neurodegenerative diseases. Preferably, the neurological disease is ischemic stroke.

The multifunctional polypeptide or its salt provided by the present invention may be prepared as medicament. The aforementioned medicament is injectable agent. Specifically, the injectable agent is powder or injectable solution. Further, the aforementioned medicament is administered intravenously. The aforementioned medicament may also be used as active ingredient to prepare other dosage forms, so as to adapt for corresponding medical applications. As a neuroprotective drug, the formulation developed may be used for oral administration and sublingual administration. As a first-aid emergency medicament for cerebral stroke, the formulation developed may be used for spray administration, anal administration and so on, so that the patients with no mobility can be treated. In a further aspect, the present invention also provides a method of preparing various synthetic peptides of the present invention by solid-phase synthesis, however, it is more convenient to use liquid-phase synthesis for some of the peptides. Salt form of the polypeptide drug is one of the common means to improve the physical and chemical properties of drug molecules as well as enhance druggability. The aforementioned drug can be any form of salt.

In order to determine the application of the synthetic peptides provided by the present invention in neurological diseases, the present invention uses SD rats as experimental objects, and performs middle cerebral artery occlusion method (MCAO) to prepare rat model of cerebral ischemia. These rats were injected drugs intravenously 1-2 hours after ischemia, and a behavioral observation and scoring was conducted for each animal after 22-24 hours. After the behavioral observation, the experimental rats were euthanized and their brains were removed, cut to sections and stained with TTC for quantitative analysis, and the cerebral infarct volume % was calculated.

The synthetic peptide of the present invention creatively combines glycine with carnosine, anserine or ophidine, etc. to develop a mosaic synthetic peptide, which unexpectedly passes the blood-brain barrier through intravenous injection, enters the brain, and plays an effective role in the treatment of neurological diseases. Such synthetic peptides are preferably used in the treatment of ischemic stroke, hemorrhagic stroke, cerebral trauma, Alzheimer's disease, Parkinson's disease and other neurodegenerative diseases. The present invention only needs 3 mg/kg intravenous injection dose of the synthetic polypeptide to surprisingly achieve remarkable therapeutic effect, which is equivalent to the effect achieved by clinically active standard polypeptide NA1.

The synthetic peptide consists of glycine and carnosine, anserine or ophidine, all of which are natural amino acids or peptides with safety and reliablity.

The synthetic peptide of the present invention is easy to synthesize. During the synthesis process, the derivatives of carnosine, anserine or ophidine may also be used as synthetic fragments to replace the corresponding two amino acids to further simplify the synthesis of the peptide of interest. In addition, some short peptides such as beta-Ala-His-Gly, Gly-beta-Ala-His, beta-Ala-His-Gly-Gly, Gly-Gly-beta-Ala-His, Gly-beta-Ala-His-Gly, beta-Ala-(1-Methyl-His)-Gly, Gly-beta-Ala-(1-Methyl-His), beta-Ala-(1-Methyl-His)-Gly-Gly, Gly-Gly-beta-Ala-(1-Methyl-His), Gly-beta-Ala-(1-Methyl-His)-Gly, beta-Ala-(3-Methyl-His)-Gly, Gly-beta-Ala-(3-Methyl-His), beta-Ala-(3-Methyl-His)-Gly-Gly, Gly-Gly-beta-Ala-(3-Methyl-His) and Gly-beta-Ala-(3-Methyl-His)-Gly can be obtained through liquid phase synthesis to further reduce the cost as well as to make large-scale production and product quality supervision easier in the future.

**Description of the drawings**

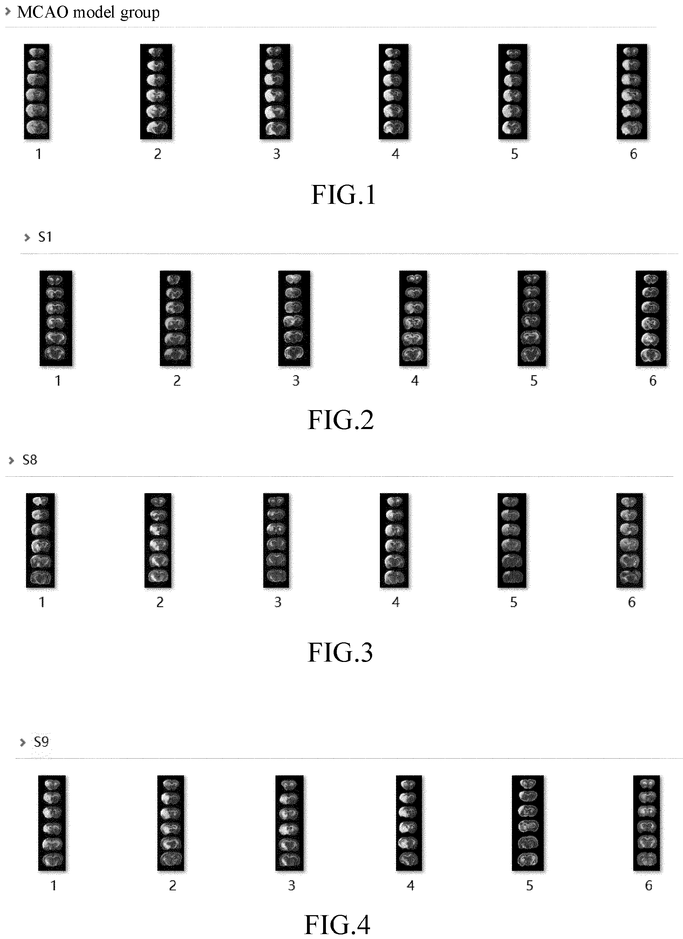

Fig. 1 shows the slice section of the brain tissue in the model group;
Fig. 2 shows the slice section of the brain tissue in S1 group;
Fig. 3 shows the slice section of the brain tissue in S8 group;
Fig. 4 shows the slice section of the brain tissue in S9 group;

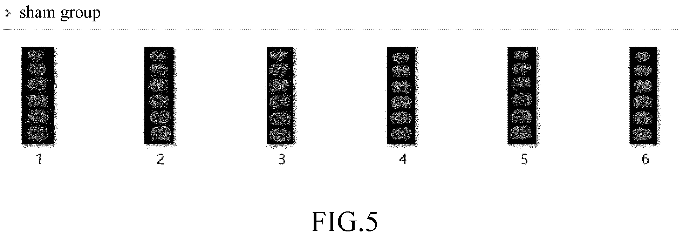

Fig. 5 shows the slice section of the brain tissue in the sham group.

**Detailed description**

In order to make the object, technical solutions and advantages of the present invention more clear, the present invention will be further described in details below in conjunction with the accompanying drawings.

Example 1: The synthesis of beta-Ala-His-Gly

1. In the presence of an activator system (HoBT, DIC), the Fmoc-Gly-CTC resin was obtained by coupling the resin solid-phase support 2-CTC resin with Fmoc-Gly-OH.
2. 20% piperidine was used to remove the Fmoc protecting group on Fmoc-Gly-CTC, which was then washed with DMF after removal.
3. 3 times of excess Fmoc-His(Trt)-OH and 3 times of excess activator was weighted, and a small amount of DMF was added to fully dissolve, the product after dissolution was added to the clean resin, and was washed with DMF after reacting for 1 hour.
4. The second step and the third step were repeated, and beta-Ala was coupled to Fmoc-His-Gly-CTC resin.
5. Cleavage was carried out after the synthesis completed. The ratio of the cleavage reagent was TFA:EDT:Tis:TA:anisole:$H_2O$=80:5:1:5:5:4 (volume ratio), and 10ml cleavage reagent was used for 1g peptide resin. The cleavage was performed at room temperature for about 2h (120 r/min), and a precipitation using ice methyl tert-butyl ether was preformed after the cleavage, the bottom precipitate was the crude product.
6. The crude peptide from previous step was dissolved and purified with 0.1% TFA/acetonitrile on preparative HPLC.
7. A freeze-dry step was performed after purification, and the powder was taken for quality examination after freeze-drying.

Example 2: The synthesis of Gly-beta-Ala-His

1. In the presence of an activator system (HoBT, DIC), the Fmoc-His(Trt)-CTC resin was obtained by coupling the solid-phase support 2-CTC resin with Fmoc-His(Trt)-OH.
2. 20% piperidine was used to remove the Fmoc protecting group on Fmoc-His(Trt)-CTC, which was then washed with DMF after removal.
3. 3 times of excess Fmoc-beta-Ala-OH and 3 times of excess activator was weighted, and a small amount of DMF was added to fully dissolve, the product after dissolution was added to the clean resin, and was washed with DMF after reacting for 1 hour.
4. The second step and the third step were repeated, and Gly was coupled to Fmoc-beta-Ala-His-CTC resin.
5. Cleavage was carried out after the synthesis completed. The ratio of the cleavage reagent was TFA:EDT:Tis:TA:anisole:$H_2O$=80:5:1:5:5:4 (volume ratio), and 10ml cleavage reagent was used for 1g peptide resin. The cleavage was performed at room temperature for about 2h (120 r/min), and a precipitation using ice methyl tert-butyl ether was preformed after the cleavage, the bottom precipitate was the crude product.
6. The crude peptide from previous step was dissolved and purified with 0.1% TFA/acetonitrile on preparative HPLC.
7. A freeze-dry step was performed after purification, and the powder was taken for quality examination after freeze-drying.

Example 3: The synthesis of Gly-beta-Ala-His-beta-Ala-His-Gly (SEQ ID NO: 1)

1. In the presence of an activator system (HoBT, DIC), the Fmoc-Gly-CTC resin was obtained by coupling the resin solid-phase support 2-CTC resin with Fmoc-Gly-OH.

2. 20% piperidine was used to remove the Fmoc protecting group on Fmoc-Gly-CTC, which was then washed with DMF after removal.

3. 3 times of excess Fmoc-His(Trt)-OH and 3 times of excess activator was weighted, and a small amount of DMF was added to fully dissolve, the product after dissolution was added to the clean resin, and was washed with DMF after reacting for 1 hour.

4. The second step and the third step were repeated, and beta-Ala, His, beta-Ala and Gly was sequentially coupled to Fmoc-His-Gly-CTC resin.

5. Cleavage was carried out after the synthesis completed. The ratio of the cleavage reagent was TFA:EDT:Tis:TA:anisole:$H_2O$=80:5:1:5:5:4 (volume ratio), and 10ml cleavage reagent was used for 1g peptide resin. The cleavage was performed at room temperature for about 2h (120 r/min), and a precipitation using ice methyl tert-butyl ether was preformed after the cleavage, the bottom precipitate was the crude product.

6. The crude peptide from previous step was dissolved and purified with 0.1% TFA/acetonitrile on preparative HPLC.

7. A freeze-dry step was performed after purification, and the powder was taken for quality examination after freeze-drying.

Example 4: The synthesis of beta-Ala-His-Gly-Gly (SEQ ID NO: 2)

1. In the presence of an activator system (HoBT, DIC), the Fmoc-Gly-CTC resin was obtained by coupling the resin solid-phase support 2-CTC resin with Fmoc-Gly-OH.

2. 20% piperidine was used to remove the Fmoc protecting group on Fmoc-Gly-CTC, which was then washed with DMF after removal.

3. 3 times of excess Fmoc-Gly-OH and 3 times of excess activator was weighted, and a small amount of DMF was added to fully dissolve, the product after dissolution was added to the clean resin, and was washed with DMF after reacting for 1 hour.

4. 20% piperidine was used to remove the Fmoc protecting group on Fmoc-Gly-Gly-CTC, which was then washed with DMF after removal.

5. 3 times of excess Fmoc-His(Trt)-OH and 3 times of excess activator was weighted, and a small amount of DMF was added to fully dissolve, the product after dissolution was added to the clean resin, and was washed with DMF after reacting for 1 hour.

6. 20% piperidine was used to remove the Fmoc protecting group on Fmoc-His(Trt)-Gly-Gly-CTC, which was then washed with DMF after removal.

7. 3 times of excess Fmoc-beta-Ala-OH and 3 times of excess activator was weighted, and a small amount of DMF was added to fully dissolve, the product after dissolution was added to the clean resin, and was washed with DMF after reacting for 1 hour.

8. 20% piperidine was used to remove the Fmoc protecting group, which was then washed with DMF after removal.

9. The ratio of the cleavage reagent was TFA:EDT:Tis:TA:anisole:$H_2O$=80:5:1:5:5:4 (volume ratio), and 10ml cleavage reagent was used for 1g peptide resin. The cleavage was performed at room temperature for about 2h (120 r/min), and a precipitation using ice methyl tert-butyl ether was preformed after the cleavage, the bottom precipitate was the crude product.

10. The crude peptide from previous step was dissolved and purified with 0.1% TFA/acetonitrile on preparative HPLC.

11. A freeze-dry step was performed after purification, and the powder was taken for quality examination after freeze-drying.

Example 5: The synthesis of Gly-Gly-beta-Ala-His (SEQ ID NO: 3)

1. In the presence of an activator system (HoBT, DIC), the Fmoc-His(Trt)-CTC resin was obtained by coupling the resin solid-phase support 2-CTC resin with Fmoc-His(Trt)-OH.

2. 20% piperidine was used to remove the Fmoc protecting group on Fmoc-His(Trt)-CTC, which was then washed with DMF after removal.

3. 3 times of excess Fmoc-beta-Ala-OH and 3 times of excess activator was weighted, and a small amount of DMF was added to fully dissolve, the product after dissolution was added to the clean resin, and was washed with DMF after reacting for 1 hour.

4. The second step and the third step were repeated, and Gly and Gly were coupled to Fmoc-His-Gly-CTC resin.

5. Cleavage was carried out after the synthesis completed. The ratio of the cleavage reagent was TFA:EDT:Tis:TA:anisole:$H_2O$=80:5:1:5:5:4 (volume ratio), and 10ml cleavage reagent was used for 1g peptide resin. The cleavage was performed at room temperature for about 2h (120 r/min), and a precipitation using ice methyl tert-butyl ether was preformed after the cleavage, the bottom precipitate was the crude product.

6. The crude peptide from previous step was dissolved and purified with 0.1% TFA/acetonitrile on preparative HPLC.

7. A freeze-dry step was performed after purification, and the powder was taken for quality examination after freeze-drying.

Example 6: The synthesis of Gly-beta-Ala-His-Gly (SEQ ID NO: 4)

1. In the presence of an activator system (HoBT, DIC), the Fmoc-Gly-CTC resin was obtained by coupling the resin solid-phase support 2-CTC resin with Fmoc-Gly-OH.

2. 20% piperidine was used to remove the Fmoc protecting group on Fmoc-Gly-CTC, which was then washed with DMF after removal.

3. 3 times of excess Fmoc-His(Trt)-OH and 3 times of excess activator was weighted, and a small amount of DMF was added to fully dissolve, the product after dissolution was added to the clean resin, and was washed with DMF after reacting for 1 hour.

4. The second step and the third step were repeated, and beta-Ala and Gly was sequentially coupled to Fmoc-His-Gly-CTC resin.

5. Cleavage was carried out after the synthesis completed. The ratio of the cleavage reagent was TFA:EDT:Tis:TA:anisole:$H_2O$=80:5:1:5:5:4 (volume ratio), and 10ml cleavage reagent was used for 1g peptide resin. The cleavage was performed at room temperature for about 2h (120 r/min), and a precipitation using ice methyl tert-butyl ether was preformed after the cleavage, the bottom precipitate was the crude product.

6. The crude peptide from previous step was dissolved and purified with 0.1% TFA/acetonitrile on preparative HPLC.

7. A freeze-dry step was performed after purification, and the powder was taken for quality examination after freeze-drying.

Example 7: The synthesis of Gly-Gly-beta-Ala-His-Gly-Gly (SEQ ID NO: 5)

1. In the presence of an activator system (HoBT, DIC), the Fmoc-Gly-CTC resin was obtained by coupling the resin solid-phase support 2-CTC resin with Fmoc-Gly-OH.

2. 20% piperidine was used to remove the Fmoc protecting group on Fmoc-Gly-CTC, which was then washed with DMF after removal.

3. 3 times of excess Fmoc-Gly-OH and 3 times of excess activator was weighted, and a small amount of DMF was added to fully dissolve, the product after dissolution was added to the clean resin, and was washed with DMF after reacting for 1 hour.

4. The second step and the third step were repeated, and His, beta-Ala, Gly and Gly were sequentially coupled to the CTC resin.

5. Cleavage was carried out after the synthesis completed. The ratio of the cleavage reagent was TFA:EDT:Tis:TA:anisole:$H_2O$=80:5:1:5:5:4 (volume ratio), and 10ml cleavage reagent was used for 1g peptide resin. The cleavage was performed at room temperature for about 2h (120 r/min), and a precipitation using ice methyl tert-butyl ether was preformed after the cleavage, the bottom precipitate was the crude product.

6. The crude peptide from previous step was dissolved and purified with 0.1% TFA/acetonitrile on preparative HPLC.

7. A freeze-dry step was performed after purification, and the powder was taken for quality examination after freeze-drying.

Example 8: The same method can also be used to synthesize the following polypeptides:

beta-Ala-His-Gly-Gly;

Gly-Gly-beta-Ala-His;

Gly-beta-Ala-His-Gly;

beta-Ala-His-beta-Ala-His-Gly;

Gly-beta-Ala-His-beta-Ala-His;

beta-Ala-His-beta-Ala-His-Gly-Gly;

Gly-Gly-beta-Ala-His-beta-Ala-His;

beta-Ala-His-beta-Ala-His-beta-Ala-His-Gly;

Gly-beta-Ala-His-beta-Ala-His-beta-Ala-His;

beta-Ala-His-beta-Ala-His-beta-Ala-His-Gly-Gly;

Gly-Gly-beta-Ala-His-beta-Ala-His-beta-Ala-His;

beta-Ala-His-beta-Ala-His-beta-Ala-His-Gly-Gly;

Gly-Gly-beta-Ala-His-beta-Ala-His beta-Ala-His;

beta-Ala-His-beta-Ala-His-beta-Ala-His-Gly-Gly-Gly;

Gly-Gly-Gly-beta-Ala-His-beta-Ala-His-beta-Ala-His;

Gly-Gly-beta-Ala-His-beta-Ala-His-beta-Ala-His-Gly;

beta-Ala-His-beta-Ala-His-beta-Ala-His-beta-Ala-His-Gly-Gly-Gly;

Gly-Gly-Gly-beta-Ala-His-beta-Ala-His-beta-Ala-His-beta-Ala-His;

Gly-Gly-beta-Ala-His-beta-Ala-His-beta-Ala-His-beta-Ala-His-Gly;

beta-Ala-(1-Methyl-His)-Gly;

Gly-beta-Ala-(1 -Methyl-His);

beta-Ala-(1 -Methyl-His)-Gly-Gly;

Gly-Gly-beta-Ala-(1-Methyl-His);

Gly-beta-Ala-(1-Methyl-His)-Gly;

beta-Ala-(1-Methyl-His)-beta-Ala-(1-Methyl-His)-Gly;

Gly-beta-Ala-(1-Methyl-His)-beta-Ala-(1-Methyl-His);

beta-Ala-(1-Methyl-His)-beta-Ala-(1-Methyl-His)-Gly-Gly;

Gly-Gly-beta-Ala-(1-Methyl-His)-beta-Ala-(1-Methyl-His);

Gly-beta-Ala-(1-Methyl-His)-beta-Ala-(1-Methyl-His)-Gly;

beta-Ala-(3-Methyl-His)-Gly;

Gly-beta-Ala-(3-Methyl-His);

beta-Ala-(3-Methyl-His)-Gly-Gly;

8

Gly-Gly-beta-Ala-(3-Methyl-His);

Gly-beta-Ala-(3-Methyl-His)-Gly;

beta-Ala-(3-Methyl-His)-beta-Ala-(3-Methyl-His)-Gly;

Gly-beta-Ala-(3-Methyl-His)-beta-Ala-(3-Methyl-His);

beta-Ala-(3-Methyl-His)-beta-Ala-(3-Methyl-His)-Gly-Gly;

Gly-Gly-beta-Ala-(3-Methyl-His)-beta-Ala-(3-Methyl-His);

Gly-beta-Ala-(3-Methyl-His)-beta-Ala-(3-Methyl-His)-Gly.

Example 9: Animal Experiment Method

Small-animal monitor: VT200 from Rongxianda Technology Co., Ltd., Shenzhen, China.
SD rats: 180-200g, from China National Institutes for Food and Drug Identification.
Suture emboli: Maiyue Bio (M8507).
TTC staining solution: Yuanye Bio (R24053).
Animal model surgery steps:
The experimental SD rat was weighed and anesthetized with chloral hydrate. After anesthesia, the limbs of the rat were fixed, and the rat was placed in a supine position, connected to the small-animal monitor, and monitored for important physiological parameters such as body temperature, blood pressure and heart rate, etc.. The rat's neck was shaved and disinfected with 75% alcohol cotton ball. A middle incision of about 2 cm length was then made on the neck of the experimental rat, and the submandibular gland of the rat was bluntly separated while avoiding damage to the gland. Then, the left common carotid artery (CCA) of the experimental rat was bluntly separated, and the internal carotid artery (ICA) and external carotid artery (ECA) were carefully separated upward along the CCA. Any damage to vagus nerves was avoided during the separation. The CCA and ICA were clamped respectively with two artery clamps, and the distal end of the ECA was cut off and a silicone suture emboli was inserted into the ECA "port" to the ICA artery clamp, then the artery clamp was quickly loosened and the tip of the suture emboli was put in through the artery clamp quickly before clamping the ICA again. This step was repeated and the suture emboli was put in further until the black mark of the suture emboli reached the bifurcation of the ECA and ICA, and the tip of the suture emboli blocked the middle cerebral artery and then the ECA "port" and the suture emboli was tied tightly using a thread in order to prevent the "escape" of suture emboli or bleeding after the rat waked up. The artery clamps on the CCA and ICA were then loosed and removed to restore the tissue to its original position, and an appropriate amount of penicillin was applied to prevent wound infection. A medical suture needle with thread was used to suture, and the wound was disinfected using povidone-iodine again after the suturation. The experimental rats were monitored with the small-animal monitor to evaluate if their physiological parameters were normal, and the rats were placed on a small-animal electric blanket to keep their body temperature until they waked up, and placed in animal cages.
Dosing procedure:
The drug to be tested was prepared as 3mg/mL solution. The drug was dosed via tail vein 1-2h after the suturation.

During the drug administration, the rat was fastened with a fastening device, and the drug to be tested was injected into the tail vein of the rat at 3 mg/kg using 1 mL syringe, the drug was injected slowly to reduce the cardiopulmonary load of the experimental rat.
The animals were divided into different test groups, with 6 rats for each group.

Model group: After the suturation, an equal amount of normal saline was given via tail vein;
Drug groups S1 (NA1), S8 (beta-Ala-His-Gly) and S9 (Gly-beta-Ala-His): administered via tail vein after the suturation;
Sham group: the internal carotid artery (ICA) and external carotid artery (ECA) were separated without the suturation thereafter, and an equal amount of normal saline was given via tail vein.
Behavioral observation:

The behavioral observation and scoring were performed on each animal 22-24h after the suturation.

The technicians who did not participate in the above experimental procedure were asked to perform the scoring blindly according to the scoring scale below.

0 point: walking in a normal straight line;
1 point: forelimb weakness;
2 points: hindlimb weakness;
3 points: mild circling;
4 points: severe circling;
5 points: hemiplegia.

TTC staining and quantitative analysis:
After behavioral observations, experimental rats were euthanized and their brains were removed. The brain tissue was transversely cut into 6 slices with a 2mm thickness, then transferred to TTC staining solution, incubated in a 37°C incubator without light for 10min, and taken pictures. The TTC-stained brain tissue and the remaining non-stained brain tissue were stored at -20°C.
Quantitative analysis of photos after TTC staining was performed using Image J software.

$$\text{Cerebral infarction volume } \% = (\text{total infarct area} * \text{slice thickness}) / (\text{total brain slice area} * \text{slice thickness}) * 100\%.$$

Experimental results:

1.1. TTC quantification results are shown in Table 1:

Table 1

| No. | 1 | 2 | 3 | 4 | 5 | 6 | Mean value |
|---|---|---|---|---|---|---|---|
| model group | 0.407 | 0.402 | 0.392 | 0.420 | 0.377 | 0.340 | 0.390 |
| S1(NA1) | 0.187 | 0.218 | 0.153 | 0.145 | 0.107 | 0.134 | 0.157 |
| S8 | 0.236 | 0.172 | 0.086 | 0.197 | 0.150 | 0.191 | 0.172 |
| S9 | 0.239 | 0.206 | 0.208 | 0.263 | 0.171 | 0.095 | 0.197 |
| Sham group | 0.048 | 0.038 | 0.042 | 0.051 | 0.039 | 0.044 | 0.044 |

As shown by the quantification results after TTC in Table 1, the rats with cerebral ischemia injected with normal saline had an infarct volume of $0.390 \pm 0.028$; the rats with cerebral ischemia injected with 3mg/kg beta-Ala-His-Gly (S8) had an infarct volume of $0.172 \pm 0.051$; the rats with cerebral ischemia injected with 3mg/kg Gly-beta-Ala-His (S9) had an infarct volume of $0.197 \pm 0.059$; and as a comparison, the rats with cerebral ischemia injected with 3mg/kg NA1 had an infarct volume of $0.157 \pm 0.040$. As a control, the sham group had an infarct volume of $0.044 \pm 0.005$. Compared with normal saline, the other three groups had significant biological activities and S8 and S9 were not statistically different from S1. Wherein, NA1 (Nerinetide) is a neuroprotective agent (Michael Tymianski and Jonathan D. Garman. Model systems and treatment regimes for treatment of neurological disease. 2015, US Patent, US8940699B2), which can interfere with postsynaptic density protein 95 (PSD-95) by terminating the production of intracellular NO free radicals. In preclinical ischemic stroke model, NA1 can reduce the infarct size of experimental cerebral ischemia-reperfusion in animals (cynomolgus monkeys), and improve the functional prognosis, therefore is a good positive control. Dr. Hill studied the efficacy and safety of intravenous injection of a novel neuropeptide NA-1 (2.6 mg/kg) in patients with acute ischemic stroke (AIS) undergoing endovascular thrombectomy, and showed that the treatment with NA1 improved patient's prognosis. (Michael D Hill et al. Efficacy and safety of nerinetide for the treatment of acute ischaemic stroke (ESCAPE-NA1): a multicentre, double-blind, randomised controlled trial. Lancet. Published online February 20, 2020).

1.2 The behavioral scoring results are shown in Table 2:

Table 2

| No. | 1 | 2 | 3 | 4 | 5 | 6 | Mean value | Standard deviation |
|---|---|---|---|---|---|---|---|---|
| model group | 4 | 4 | 3 | 5 | 4 | 3 | 3.8 | 0.8 |
| S1 | 2 | 2 | 2 | 2 | 1 | 3 | 2.0 | 0.6 |
| S8 | 3 | 2 | 1 | 2 | 2 | 3 | 2.2 | 0.8 |
| S9 | 3 | 3 | 2 | 2 | 1 | 1 | 2.0 | 0.9 |
| Sham group | 0 | 0 | 0 | 0 | 0 | 0 | 0.0 | 0.0 |

As shown in Table 2, the synthetic peptides of the present invention have physiological activity. the rats with cerebral ischemia injected with normal saline had a behavioral score of 3.8±0.8; the rats with cerebral ischemia injected with 3mg/kg beta-Ala-His-Gly had a behavioral score of 2.2±0.8; the rats with cerebral ischemia injected with 3mg/kg Gly-beta-Ala-His had a behavioral score of 2.0±0.9; and as a comparison, the rats with cerebral ischemia given 3 mg/kg NA1 intravenously had a behavioral score of 2.0±0.6. As a control, the sham group had a behavioral score of 1.0±0.0. Compared with normal saline, the other three groups had significant biological activities, and the result of S8 and S9 were not statistically different from S1.

Example 10: The dose-effect experiment

Preparation method: an amount of test substance was weighted and fully dissolved using water for injection to the required concentration, and was filtered with a 0.22 μm filter to sterilize. Taking 280 g×14 animals in each group as an example, the specific information is shown in Table 3 below.

Table 3

| Group | Dose (mg/kg) | Concentration (mg/mL) | Preparation method |
|---|---|---|---|
| S8 low dose group | 0.6 | 0.1 | Weigh 2.352 mg of S8, add 23.52 mL of water for injection, fully dissolve and filter with a 0.22 μm filter |
| S8 medium dose group | 3 | 0.5 | Weigh 11.76 mg of S8, add 23.52 mL of water for injection, fully dissolve and filter with a 0.22 μm filter |
| S8 high dose group | 15 | 2.5 | Weigh 58.8 mg S8, add 23.52 mL water for injection, fully dissolve and filter with 0.22 μm filter |
| Negative control (vehicle): water for injection. Positive control: S1 (NA1). | | | |

Preparation method: an amount of test substance was weighted (compound purity is calculated as 100%), prepared to the required concentration, and filtered with a 0.22 μm filter to sterilize. Taking 280 g×14 animals as an example, see Table 4 below for specific information.

Table 4

| Group | Dose (mg/kg) | Concentration (mg/mL) | Preparation method |
|---|---|---|---|
| S1 | 3 | 0.5 | Weigh 11.76 mg S1, add 23.52 mL water for injection, fully dissolve and filter with 0.22 μm filter |

Animal strain: Sprague-Dawley (SD) rats; grade: SPF grade. Body weight: 200.01-240.03 g when the accommodation started, 213.79-278.79 g when grouping. Age: about 5-7 weeks old during the accommodation, and 6-8 weeks old when grouping. Source: SPF (Beijing) Biotechnology Co., Ltd., Beijing, China.
Animals were accommodated to the environment for 5 days after received; unqualified animals were excluded from this experiment.

The condition standard for breeding environment: National Standard GB14925-2010 of the People's Republic of China;

The control system for breeding environment: WINCC7.3 EMS series computer room environment monitoring system;

Temperature: room temperature 20~26 °C (daily temperature difference < 4 °C);

Relative humidity: 40 ~ 70%;

Lighting: artificial lighting, 12/12 hrs alternating light and dark;

Air exchange: no less than 15 times per hour.

Food: Rat and mouse breeding feed;

Food Production Company: Beijing Keao Xieli Feed Co., Ltd., Beijing, China;

Feeding method: free intake;

Drinking water: drinking water for experimental animals (reverse osmosis water);

Water supply method: using drinking water bottle, free intake;

Routine index testing of water quality: According to the relevant requirements of the national standard GB5749-2006 of the People's Republic of China, a qualified third-party industry was entrusted to test the water quality at least every six months.

Animal selection: Animals that passed the examination during the accommodation, whose body weight met the experimental requirements and total NSS score less than or equal to 3 were chosen as test animals.

The establishment of rat model: the rats were placed in an anesthesia induction box filled with 3.0% isoflurane for anesthesia induction. Anesthesia-induced animals were transferred to the operating table, and anesthesia was maintained with 2.0%~2.5% isoflurane at 200 mL/min using a small-animal gas anesthesia machine, and the eyelid reflex and pain response of the rats were observed. The surgery can be started only after the eyelid reflex response and the pain response on limbs and tail disappeared.

A. Isolating and exposing blood vessels: the skin of the surgical area was shaved, and the rat was placed under a surgical microscope. The skin of the rat was cut along the midline with ophthalmic scissors, with a cut of about 2 cm length. Through the right neck of the rat, the muscle tissue of the right neck was bluntly isolated and opened with a microforcep to expose the right common carotid artery (CCA), and then isolated upward along the common carotid artery to further expose the external carotid artery (ECA) and internal carotid artery (ICA);

B. The ECA was ligated and the ICA was temporarily clamped; the proximal end and distal end of CCA were threaded separately, the distal end was tied tightly and the proximal end was tied with a loose knot, and a small incision was made between the two threads;

C. Inserting the suture emboli: A No. 4-0 suture emboli was inserted through the incision of CCA, pushed slowly and gently into the internal carotid artery, and stopped at the artery clamp of ICA, the pre-set thread was tied tightly (to avoid severe bleeding when pushing the suture emboli), then the artery clamp that blocked the blood flow of ICA was removed, and the suture emboli was immediately pushed into ICA until entering into the cranium. At the moment, one should be careful not to insert the suture emboli into the pterygopalatine artery, which is another branch of the internal carotid artery (the pterygopalatine artery is one of the extra-cranial branches of ICA; when the suture emboli entered the pterygopalatine artery to a depth of about 10 mm, it cannot be inserted further, at the moment one should slightly draw back the suture emboli, adjust the direction, and insert again);

D. Fixing the suture emboli and suturing the incision: if one felt slight resistance when the suture emboli was inserted about 18 mm depth from the bifurcation of the common carotid artery, it means that the tip of the suture emboli has entered into the cerebral anterior artery (ACA), and the side wall of the suture emboli has blocked the opening of the middle cerebral artery; at the moment, the insertion was terminated and the time was recorded. The artery clamp on CCA was removed, and the incision was closed after there was no active bleeding observed;

E. Performing cerebral reperfusion: the ischemic rat was placed at room temperature, and the anesthesia was induced 120 mins thereafter; the suture emboli was slowly and lightly drawn to pull the tip of suture emboli back into the external carotid artery while maintaining anesthesia, in order to perform the middle cerebral artery reperfusion;

F. Disinfecting the incision with povidone-iodine.

Group design: model control group, positive drug treatment group, S8 low-dose group, S8 medium-dose group, S8 high-dose group;

Sex ratio: according to what has been reported by JW Simpkins ( Simpkins JW, Rajakumar G, Zhang YQ, Simpkins CE, Greenwald D, Yu CJ, Bodor N, Day AL (1997) Estrogens may reduce mortality and ischemic damage caused by middle cerebral artery occlusion in the female rat. J Neurosurg 87:724-730) and RL Roof (Roof RL, Duvdevani R, Braswell L, Stein DG (1994) Progesterone facilitates cognitive recovery and reduces secondary neuronal loss caused by cortical contrusion injury in male rats. Exp Neurol 129: 64-69), estrogen and progesterone have neuroprotective effects on ischemic cerebral infarction in adult rats. In order to exclude the influence of estrogen and progesterone on experimental results, all the animals in this experiment were male; Grouping method: random grouping was performed according to the latest body weight of rats before grouping.

See the Table 5 below for specific grouping information.

**Table 5: Dosing regimen and grouping information table**

| group | drug | Dosage (mg/kg) | Dosing concentration (mg/mL) | Dosing volume (mL/kg) | Administration route | Animal number |
|---|---|---|---|---|---|---|
| model control group | Water for Injection | - | - | 6 | i.v. | 1M001~1M014 |
| positive drug control group | S1 | 3 | 0.5 | 6 | i.v. | 2M001-2M014 |
| S8 low dose group | S8 | 0.6 | 0.1 | 6 | i.v. | 3M001-3M014 |
| S8 medium dose group | | 3 | 0.5 | 6 | i.v. | 4M001-4M014 |
| S8 high dose group | | 15 | 2.5 | 6 | i.v. | 5M001-5M014 |

Route of Administration: Intravenous injection (iv);
Dosing frequency and cycle: dose once immediately (within 10 min) after reperfusion, and the day of surgery is defined as D1 (Day1).

Animals observed: all the surviving experimental animals planned to be observed;
Observation time: once a day, and the frequency can be increased if the animal starts to behave abnormal;

What was observed: including but not limited to general manifestations, behavioral status, eye, mouth, nose and mouth area, ear, hair and skin, feces, urine, genital organ and other toxic symptoms. A detailed description was required if there was any abnormality.

Animals measured: all the surviving experimental animals planned to be measured;
Measurement time: the body weight was measured at least twice during the accommodation, and each batch of rats were weighed once on the day before the surgery and on the day of dissection to calculate the amount of drug administered and the anesthetic applied; the body weight was measured twice a week after the surgery.

**Bederson score**

Measurement time: 1 hour after ischemia on the day of modeling;
Animals measured: all the animals survived in surgery;
Measurement method: the Bederson scoring standard was used to score animals.

**Table 6: Bederson Scoring Standard for Rats**

| Score | Scoring standard |
|---|---|
| 0 | no symptom of nerve damage |
| 1 | The rat was lifted from the tail, and the forelimb on the side opposite to the surgery happened was flexed |
| 2 | The rat was placed on a smooth surface, and its shoulder on the opposite side of the infarction was pushed by hand, and the resistance decreased |

(continued)

| Score | Scoring standard |
|---|---|
| 3 | The animal fell to the opposite side of the infarction or walked in circles when walking freely |
| 4 | Paraplegia, no spontaneous movement of limbs |

Note: the rats with a score of greater than or equal to 1 after ischemia and reperfusion were successfully modeled, and the rats that failed the modeling were excluded.

**Rat neurological function scoring**

Measurement time: 24 hours before and after modeling;
Animals measured: all the surviving experimental animals planned to be measured;
Measurement method: a motor function test, sensory test, balance test, and reflex and abnormal behavior test were performed on animals, according to the rat neurological function score scale (NSS) for details.

**Table 7: Rat Neurological Function Scoring Scale (NSS)**

| Classification | Score |
|---|---|
| **Motor function test (6 points)** | |
| Lift the rat by its tail (3 points) | |
| Forelimb flexion | 1 |
| Hindlimb flexion | 1 |
| Head tilted more than 10 degrees within 30 seconds | 1 |
| Put the rat on the platform (normal 0 points, maximum 3 points) | |
| Walk normally | 0 |
| Unable to crawl straight | 1 |
| Walk in circles around the hemiplegic side | 2 |
| Fall to the hemiplegic side | 3 |
| **Sensory test (2 points)** | |
| Placement experiment (visual and tactile test) | 1 |
| Proprioception testing (deep sensation, the affected limb was placed on edge of table and observed for limb contraction response) | 1 |
| **Balance test (6 points)** | |
| Grab one side of the rod | 1 |
| Hold on to the rod, with a limb drops | 2 |
| Hold on to the rod with both limbs drop or hold on to the rod and rotate for >60 seconds | 3 |
| Try to balance but fail and fall (>40 seconds) | 4 |
| Try to balance but fail and fall (>20 seconds) | 5 |
| Fall (<20 seconds) | 6 |
| **Reflex and abnormal behavior test (4 points)** | |
| Pinna reflection | 1 |
| Corneal reflection | 1 |
| Startle reflection | 1 |
| Convulsion, myoclonus, dystonia | 1 |

**Pathological (TTC staining) evaluation**

Examination method: the animal was euthanized, the brain was quickly removed and placed in a -20°C refrigerator until the brain tissue became hard, then the rat brain was taken out and cut into 2 mm thick slices, and a total of 6 slices from the olfactory bulb to the back were placed in a six-well plate filled with 0.5% TTC solution (prepared with 0.9% sodium chloride injectable solution), incubated in a 37°C incubator without light for 20 min, and then stored in 4% formaldehyde solution without light.

The normal tissue was stained rose red, and infarcted tissue was stained white. Each brain section was placed in order on a filter paper, and taken pictures using digital camera, and the white tissue was carefully removed and weighed. The infarct size (%) was calculated as the percentage of the infarct tissue weight to the whole brain weight. The inhibition rate (%) of each drug treatment group was calculated based on the infarct size, and the inhibition rate calculation formula was as follows:

$$\text{Inhibition rate }(\%) = \frac{\text{the infract size of model group} - \text{the infract size of treatment group}}{\text{the infract size of model group}} \times 100\%.$$

Statistical analysis:

The measurement results were expressed as mean ± standard deviation. The data from a group will not be included in the statistical comparison if the sample number of this group was less than 3.

The data were input and statistically analyzed by Excel 2010, GraphPad Prism 7, SPSS 22.0 and Stata 15.0 software. Firstly, the LEVENE variance homogeneity test was used for measurement. When the variance was homogeneous (p>0.05), the result from the variance analysis was directly used to determine whether the overall difference was statistically significant. When the overall difference was statistically significant (p<0.05), the Dunnett-t test was used to compare the difference between groups. When the overall difference was not statistically significant (p>_0.05), the statistical analysis was finished. When the LEVENE variance homogeneity test showed that the variance was not homogeneous (p≤0.05), the non-parametric test (Kruskal-Wallis H test) was used. When the Kruskal-Wallis H test showed that the overall difference was statistically significant (p<0.05), the Mann-Whitney U test was used to compare the difference between groups. When the Kruskal-Wallis H test showed that the overall difference was not statistically significant (p>_0.05), the statistical analysis was finished. Detailed description of pathology and other information (if necessary) was made.

**Experimental results**

The Effect of Test Substances on the Cerebral Infarction Size in Model Animal

The drug treatment for acute ischemic stroke mainly includes two parts, one is to restore blood flow by thrombolytic therapy; the other is to administer neuroprotective agents to treat the secondary neuronal damage caused by ischemia. In this experiment, SD rats were re-perfused 120 min after ischemia to simulate vascular recanalization. Immediately (within 10 min) after the recanalization, the test substance was injected into the tail vein to induce intervention. The brain tissue was taken 24 hours after the treatment and measured by TTC staining for the infarct size of each group, and the inhibition rate was calculated to evaluate the neuroprotective effect of test substances. S1 (3 mg/kg) was used as positive control in this test. The cerebral infarction size and the inhibition rate of cerebral infarction in animals are shown in Table 8. One day after the surgery, TTC staining was performed on the brain tissue of animals to obtain the cerebral infarction size. The cerebral infarction size of the model control group and the positive control group was 24.84±3.39 % and 16.74±3.14 %, respectively, and the positive control S1 at 3 mg/kg dose showed a good effect of reducing cerebral infarction size, which was significantly different from the effect of the model control group (P<0.0001). The cerebral infarction size of the low-dose group, medium-dose group and high-dose group of test substance S8 were 20.37±4.61%, 16.94±3.90% and 15.76±2.68 %, respectively, and the test substance S8 had the effect of significantly improving cerebral tissue infarction at both 3 mg/kg and 15 mg/kg dose (P<0.0001 and P<0.0001). In addition, the inhibition rate of animal cerebral infarction in each treatment group were calculated, and the inhibition rate of animal cerebral infarction in the positive control group, test substance S8 low-dose group, S8 medium-dose group, and S8 high-dose group were 33%, 18%, 32% and 37%, respectively. The above results suggest that the test substance S8 has a good neuroprotective effect under the experimental conditions.

**Table 8: The cerebral infarction size and the inhibition rate of cerebral infarction in experimental animals**

| Group | Whole brain weight (g) | Infarct weight (g) | Infarct size (%) | Inhibition rate of cerebral infarction (%) |
|---|---|---|---|---|
| Model control group (N=13) | 1.37±0.11 | 0.34±0.06 | 24.84±3.39 | - |

(continued)

| Group | Whole brain weight (g) | Infarct weight (g) | Infarct size (%) | Inhibition rate of cerebral infarction (%) |
|---|---|---|---|---|
| Positive drug treatment group (N=14) | 1.5±0.54 | 0.24±0.02 | 16.74±3.14**** | 33 |
| S8 low dose group (N=11) | 1.37±0.08 | 0.28±0.06 | 20.37±4.61 | 18 |
| S8 medium dose group (N=14) | 1.38±0.10 | 0.23±0.06 | 16.94±3.90**** | 32 |
| S8 high dose group (N=14) | 1.42±0.06 | 0.22±0.04 | 15.76±2.68**** | 37 |

Note: The data in the table is expressed as mean ± standard deviation (Mean ± SD); "N" represents the number of animals in each group for statistical analysis, "-" indicates no data available, and * indicates P<0.05 compared to the model control group, *** indicates P<0.001 compared to the model control group, and **** indicates P<0.0001 compared to the model control group.

The main purpose of clinical treatment for cerebral infarction is to restore the patient's neurological function as much as possible and to improve the quality of life after stroke. In this experiment, the animals were evaluated with Bederson scoring 1 hour after ischemia to evaluate the ischemia in animals. One day after modeling, the neurobehavioral function of animals was evaluated with NSS scoring scale in order to evaluate the effect of test substance in improving the neurological function of animals. The Bederson scoring results of animals are shown in Table 9, and the NSS scoring results are shown in Table 10. One hour after ischemia, the Bederson score of all the groups were 3 points, suggesting that the model animals were successfully ischemic. One day after modeling, the NSS score of animals in the model control group, positive control group, test substance S8 low-dose group, S8 medium-dose group and S8 high-dose group were 8.62±0.84, 8.00±0.76, 8.36±1.61, 7.14±1.81 and 7.93±0.59, respectively. Wherein, the NSS score of the S8 medium-dose group was significantly lower than the model group, and there was a significant difference between the two groups (P<0.01). These results suggest that the test substance S8 has the effect of improving neurological function damage in stroke animals under the condition of this experiment.

**Table 9: Animal Bederson Scoring Results**

| Group | Bederson |
|---|---|
| Model control group (N=13) | 3±0 |
| Positive drug treatment group (N=14) | 3±0 |
| S8 low dose group (N=11) | 3±0 |
| S8 medium dose group (N=14) | 3±0 |
| S8 high dose group (N=14) | 3±0 |

Note: The data in the table are expressed as mean ± standard deviation (Mean ± SD); "N" represents the number of animals in each group for statistical analysis.

**Table 10: Animal NSS Scoring Results**

| Group | NSS score | |
|---|---|---|
| | D0 | D2 |
| Model control group (N=13) | 0±0 | 8.62±0.84 |
| Positive drug treatment group (N=14) | 0±0 | 8.00±0.76 |
| S5 low dose group (N=13) | 0±0 | 8.23±0.97 |
| S5 medium dose group (N=13) | 0±0 | 7.92±0.62 |
| S5 high dose group (N=13) | 0±0 | 7.85±0.66 |

Note: The data in the table are expressed as mean ± standard deviation (Mean ± SD); "N" represents the number of animals in each group for statistical analysis; "D0" represents 1 day before surgery, and "D2" represents 1 day after surgery; ** indicates P<0.01 compared with the model control group.

The effect of test substance on the weight of model animals: cerebral ischemia-reperfusion is an acute injury model, wherein the weight of experimental animals will drop dramatically after MCAO modeling. Thus, the weight of experimental animals is one of the most basic sensitive parameters to comprehensively reflect the health status of animals. In this experiment, the body weight of all the animals was monitored from first day after the surgery, and the results of animal's body weight change are shown in Table 11. There was no difference in the weight of the animals in each group before modeling. One day after the surgery, the weight of the animals in each group decreased significantly, wherein the animal weight of the model control group, the positive control group, the test substance S5 low-dose group, S5 medium-dose group and S5 high-dose group decreased by 48.92±5.78 g, 48.22±8.23 g, 42.73±8.59 g, 48.83±5.18 g, and 46.66±10.90 g, respectively, and there was no significant difference in weight loss between these groups. The above results indicate that the positive control S1 and the test substance S5 have no significant influence on rat's body weight.

**Table 11: Body weight changes of surviving animals**

| Group | Weight (g) | | |
| --- | --- | --- | --- |
| | D0 | D2 | Weight Loss |
| Model control group (N=13) | 251.16±8.66 | 202.25±7.04 | 48.92±5.78 |
| Positive drug treatment group (N=14) | 251.91±8.69 | 203.7±10.94 | 48.22±8.23 |
| S8 low dose group (N=11) | 0±0 | 8.36±1.61 | S8 low dose group(N=11) |
| S8 medium dose group (N=14) | 0±0 | 7.14±1.81** | S8 medium dose group(N=14) |
| S8 high dose group (N=14) | 0±0 | 7.93±0.59 | S8 high dose group(N=14) |

Note: The data in the table are expressed as mean ± standard deviation (Mean ± SD); "N" represents the number of animals in each group for statistical analysis; "D0" represents 1 day before surgery, "D2" represents 1 day after surgery, body weight loss = D0 body weight - D2 body weight.

This experiment uses the classic suture-embolized MCAO model to evaluate the neuroprotective effect of test substance S8. The test substance was given to animals immediately (within 10 min) after reperfusion in order to induce intervention, and 1 day after treatment, the cerebral infarct size and the inhibition rate by TCC staining, the NSS score, the grid walking test and the body weight change were measured in order to evaluate the neuroprotective effect of the test substance on rat cerebral ischemic stroke. The results showed that:

1) The test substance S8 has a good neuroprotective effect, which is mainly reflected by that the cerebral infarction size in model animals was significantly reduced after the treatment with test substance. S8 started to show significant therapeutic effect at a dose of 3 mg/kg.
2) The test substance S8 can repair the neural function damage in MCAO animals. After intervened by the test substance S8 (3 mg/kg), the NSS score of the animals decreased significantly compared to the model control group.
3) The test substance S8 had no significant effect on the body weight of the cerebral stroke rats. On the first day after the surgery, the body weight of the model animals decreased significantly, and there was no significant difference in the amount of body weight loss between the groups.

In summary, the experiment successfully established an animal model suitable for evaluating the pharmacology in ischemic cerebral stroke, and the drug efficacy evaluation results on this animal model showed that the test substance S8 has a significant therapeutic effect on ischemic cerebral stroke.

The above descriptions are only preferred embodiments of the present invention, and are not intended to limit the present invention. Any modifications, equivalent replacements, improvements, etc. made within the spirit and principles of the present invention shall be included in the protection scope of the present invention.

## Claims

1. A synthetic peptide with a chemical formula of:

(Gly)x-(beta-Ala-His)y-(Gly)z

or

(Gly)x-(beta-Ala-1-Methyl-His)y-(Gly)z

or

(Gly)x-(beta-Ala-3-Methyl-His)y-(Gly)z;

in the formula, x is an integer between 0-3, y is an integer between 1-3, and z is an integer between 0-3, and x and z are not both equal to zero.

2. The synthetic peptide according to claim 1, wherein its chemical formula may be selected from any of:

      (a) beta-Ala-His-Gly;
      (b) Gly-beta-Ala-His;
      (c) beta-Ala-His-Gly-Gly;
      (d) Gly-Gly-beta-Ala-His;
      (e) Gly-beta-Ala-His-Gly;
      (f) Gly-Gly-beta-Ala-His-Gly-Gly;
      (g) beta-Ala-His-beta-Ala-His-Gly;
      (h) Gly-beta-Ala-His-beta-Ala-His;
      (i) beta-Ala-His-beta-Ala-His-Gly-Gly;
      (j) Gly-Gly-beta-Ala-His-beta-Ala-His;
      (k) Gly-beta-Ala-His-beta-Ala-His-Gly;
      (l) Gly-Gly-beta-Ala-His-beta-Ala-His-beta-Ala-His-Gly;
      (m) beta-Ala-His-beta-Ala-His-beta-Ala-His-Gly-Gly-Gly;
      (n) Gly-Gly-Gly-beta-Ala-His-beta-Ala-His-beta-Ala-His;
      (o) Gly-beta-Ala-His-beta-Ala-His-beta-Ala-His-Gly-Gly.

3. The synthetic peptide according to claim 1, wherein its chemical formula may be selected from any of:

      (a) beta-Ala-(1-Methyl-His)-Gly;
      (b) Gly-beta-Ala-(1-Methyl-His);
      (c) beta-Ala-(1-Methyl-His)-Gly-Gly;
      (d) Gly-Gly-beta-Ala-(1-Methyl-His);
      (e) Gly-beta-Ala-(1-Methyl-His)-Gly;
      (f) Gly-Gly-beta-Ala-(1-Methyl-His)-Gly-Gly;
      (g) beta-Ala-(1-Methyl-His)-beta-Ala-(1-Methyl-His)-Gly;
      (h) Gly-beta-Ala- 1-Methyl-His-beta-Ala-(1-Methyl-His);
      (i) beta-Ala-(1-Methyl-His)-beta-Ala-(1-Methyl-His)-Gly-Gly;
      (j) Gly-Gly-beta-Ala-(1-Methyl-His)-beta-Ala-(1-Methyl-His);
      (k) Gly-beta-Ala-(1-Methyl-His)-beta-Ala-(1-Methyl-His)-Gly;
      (l) Gly-Gly-beta-Ala-(1-Methyl-His)-beta-Ala-(1-Methyl-His)-beta-Ala-(1-Methyl-His)-Gly;
      (m) beta-Ala-(1-Methyl-His)-beta-Ala-(1-Methyl-His)-beta-Ala-(1-Methyl-His)-Gly-Gly-Gly;
      (n) Gly-Gly-Gly-beta-Ala-(1-Methyl-His)-beta-Ala-(1-Methyl-His)-beta-Ala-(1-Methyl-His);
      (o) Gly-beta-Ala-(1-Methyl-His)-beta-Ala-(1-Methyl-His)-beta-Ala-(1-Methyl-His)-Gly-Gly.

4. The synthetic peptide according to claim 1, wherein its chemical formula may be selected from any of:

      (a) beta-Ala-(3-Methyl-His)-Gly;
      (b) Gly-beta-Ala-(3-Methyl-His);
      (c) beta-Ala-(3-Methyl-His)-Gly-Gly;
      (d) Gly-Gly-beta-Ala-(3-Methyl-His);
      (e) Gly-beta-Ala-(3-Methyl-His)-Gly;
      (f) Gly-Gly-beta-Ala-(3-Methyl-His)-Gly-Gly;
      (g) beta-Ala-(3-Methyl-His)-beta-Ala-(3-Methyl-His)-Gly;
      (h) Gly-beta-Ala-(3-Methyl-His)-beta-Ala-(3-Methyl-His);
      (i) beta-Ala-(3-Methyl-His)-beta-Ala-(3-Methyl-His)-Gly-Gly;
      (j) Gly-Gly-beta-Ala-(3-Methyl-His)-beta-Ala-(3-Methyl-His);
      (k) Gly-beta-Ala-(3-Methyl-His)-beta-Ala-(3-Methyl-His)-Gly;
      (l) Gly-Gly-beta-Ala-(3-Methyl-His)-beta-Ala-(3-Methyl-His)-beta-Ala-(3-Methyl--His)-Gly;
      (m) beta-Ala-(3-Methyl-His)-beta-Ala-(3-Methyl-His)-beta-Ala-(3-Methyl-His)-Gly-Gly-Gly;

(n) Gly-Gly-Gly-beta-Ala-(3-Methyl-His)-beta-Ala-(3-Methyl-His)-beta-Ala-(3-Methyl-His);

(o) Gly-beta-Ala-(3-Methyl-His)-beta-Ala-(3-Methyl-His)-beta-Ala-(3-Methyl-His)-Gly-Gly.

5. The synthetic peptide according to claim 1, wherein its chemical formula may be selected from any of:

(a) beta-Ala-His-Gly;
(b) Gly-beta-Ala-His;
(c) beta-Ala-His-Gly-Gly;
(d) Gly-Gly-beta-Ala-His;
(e) Gly-beta-Ala-His-Gly;
(f) Gly-Gly-beta-Ala-His-Gly-Gly.

6. The synthetic peptide according to claim 1, wherein its chemical formula may be selected from any of:

(a) beta-Ala-His-Gly;
(b) Gly-beta-Ala-His.

7. The synthetic peptide according to any one of claims 1-6 or a salt thereof for use in medicine.

8. The synthetic peptide according to any one of claims 1-6 or a salt thereof, for use in the treatment of ischemic stroke, hemorrhagic stroke, cerebral trauma, Alzheimer's disease, Parkinson's disease or other neurodegenerative diseases.

9. A medicament comprising the synthetic peptide according to any one of claims 1-6 or a salt thereof, wherein the medicament is administered by injection, oral, sublingual, spray or anal route, for example.

10. The medicament according to claim 9, wherein the medicament is in the form of injectable powder or injectable solution.

MCAO model group

FIG.1

S1

FIG.2

S8

FIG.3

S9

FIG.4

sham group

FIG.5

## INTERNATIONAL SEARCH REPORT

| | International application No. |
|---|---|
| | **PCT/CN2022/080445** |

| A. | CLASSIFICATION OF SUBJECT MATTER |
|---|---|

C07K 5/083(2006.01)i; A61K 38/06(2006.01)i; A61K 38/07(2006.01)i; A61P 25/16(2006.01)i; A61P 9/10(2006.01)i; A61P 7/04(2006.01)i; C07K 7/06(2006.01)i; C07K 5/103(2006.01)i; A61K 38/08(2019.01)i; A61P 25/28(2006.01)i; A61P 25/00(2006.01)i

According to International Patent Classification (IPC) or to both national classification and IPC

| B. | FIELDS SEARCHED |
|---|---|

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CNABS; CNMED; CNTXT; VEN; DWPI; SIPOABS; EPTXT; WOTXT; USTXT; JPTXT; CNKI; 中国专利生物序列检索系统, China Patent Biological Sequence Retrieval System; Genbank; NCBI; EMBL; STN; Elsevier; WEB OF SCIENCE; Pubmed 和检索项, Pubmed and search terms: 甘氨酸, 氨基乙酸, 肌肽, 鹅肌肽, 蛇肌肽, Glycerine, Gly, Carnosine, Anserine, goose carnosine, Balenine, Ophidine, snake carnosine, beta 1W Ala 1W His, beta 1W Ala 1W Methyl 1W His.

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 113150065 A (WUHAN INNERSE PHARMACEUTICALS INC.) 23 July 2021 (2021-07-23)<br>claims 1-10 | 1-10 |
| A | CN 1579543 A (JIANG WEISHI) 16 February 2005 (2005-02-16)<br>see entire document | 1-10 |
| A | IN 201821042054 A (CIAN-N CIAN HEALTH CARE PVT LTD) 08 May 2020 (2020-05-08)<br>see entire document | 1-10 |
| A | 沈耀等 (SHEN, Yao et al.). "肌肽和中枢神经系统疾病 (Carnosine and Diseases of Central Nervous System)"<br>浙江大学学报 (医学版) (Journal of Zhejiang University (Medical Sciences)),<br>Vol. 36, No. 2, 25 March 2007 (2007-03-25),<br>pp. 199-203, see entire document | 1-10 |

☑ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **22 May 2022** | **09 June 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2022/080445** |

### C.    DOCUMENTS CONSIDERED TO BE RELEVANT

| Category* | Citation of document, with indication, where appropriate, of the relevant passages | Relevant to claim No. |
| --- | --- | --- |
| A | 沈杰等 (SHEN, Jie et al.). "肌肽在医药方面的研究进展 (Research Advances of Application of Carnosine in Pharmaceutical Field)" <br> 中国生化药物杂志 (Chinese Journal of Biochemical Pharmaceutics), <br> Vol. 29, No. 2, 20 April 2008 (2008-04-20), <br>       pp. 134-137, see entire document | 1-10 |
| A | CHMIELEWSKA, K. et al. "Therapeutic Potential of Carnosine and Its Derivatives in the Treatment of Human Diseases" <br> Chemical Research in Toxicology, Vol. 33, No. 7, 17 April 2020 (2020-04-17), <br>       pp. 1561-1578, see entire document | 1-10 |

Form PCT/ISA/210 (second sheet) (January 2015)

**INTERNATIONAL SEARCH REPORT**

| International application No. |
| --- |
| **PCT/CN2022/080445** |

**Box No. II**      **Observations where certain claims were found unsearchable (Continuation of item 2 of first sheet)**

This international search report has not been established in respect of certain claims under Article 17(2)(a) for the following reasons:

1. ☑   Claims Nos.: **8**
   because they relate to subject matter not required to be searched by this Authority, namely:

   [1]   PCT Rule 39.1(iv)-methods for the treatment of the human or animal body by surgery or therapy.

   [2]   The search and the written opinion for claim 8 are made on the basis that it is reasonably expected that the claimed subject matter is a use of the synthetic peptide or a salt thereof according to any one of claims 1-6 in the preparation of a drug for treating ischemic stroke, hemorrhagic stroke, traumatic brain injury, Alzheimer's disease, Parkinson's disease, or other neurodegenerative diseases.

2. ☐   Claims Nos.:
   because they relate to parts of the international application that do not comply with the prescribed requirements to such an extent that no meaningful international search can be carried out, specifically:

3. ☐   Claims Nos.:
   because they are dependent claims and are not drafted in accordance with the second and third sentences of Rule 6.4(a).

Form PCT/ISA/210 (continuation of first sheet) (January 2015)

## INTERNATIONAL SEARCH REPORT
**Information on patent family members**

International application No.

**PCT/CN2022/080445**

| Patent document cited in search report | | | Publication date (day/month/year) | Patent family member(s) | Publication date (day/month/year) |
|---|---|---|---|---|---|
| CN | 113150065 | A | 23 July 2021 | None | |
| CN | 1579543 | A | 16 February 2005 | None | |
| IN | 201821042054 | A | 08 May 2020 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8940699 B2, Michael Tymianski and Jonathan D. Garman.

**Non-patent literature cited in the description**

- Efficacy and safety of nerinetide for the treatment of acute ischaemic stroke (ESCAPE-NA1): a multicentre, double-blind, randomised controlled trial. **MICHAEL D HILL et al.** Lancet. Published, 20 February 2020
- **SIMPKINS JW ; RAJAKUMAR G ; ZHANG YQ ; SIMPKINS CE ; GREENWALD D ; YU CJ ; BODOR N ; DAY AL.** Estrogens may reduce mortality and ischemic damage caused by middle cerebral artery occlusion in the female rat. *J Neurosurg,* 1997, vol. 87, 724-730
- **ROOF RL ; DUVDEVANI R ; BRASWELL L ; STEIN DG.** Progesterone facilitates cognitive recovery and reduces secondary neuronal loss caused by cortical contrusion injury in male rats. *Exp Neurol,* 1994, vol. 129, 64-69